(19) Europäisches Patentamt
European Patent Office
Office européen des brevets

(11) Veröffentlichungsnummer: **0 082 217**
**A1**

(12) # EUROPÄISCHE PATENTANMELDUNG

(21) Anmeldenummer: **81110735.8**

(22) Anmeldetag: **23.12.81**

(51) Int. Cl.³: **C 07 D 417/12**
A 61 K 31/54

(43) Veröffentlichungstag der Anmeldung:
**29.06.83 Patentblatt 83/26**

(84) Benannte Vertragsstaaten:
**AT BE CH DE FR IT LI LU NL SE**

(71) Anmelder: **CIBA-GEIGY AG**
**Patentabteilung Postfach**
**CH-4002 Basel(CH)**

(72) Erfinder: **Sele, Alex**
**Langmattstrasse 14**
**CH-4132 Muttenz(CH)**

(72) Erfinder: **Ferrini, Pier Giorgio, Dr.**
**Im Rehwechsel 22**
**CH-4102 Binningén(CH)**

(72) Erfinder: **Haas, Georges, Dr.**
**Im Rehwechsel 24**
**CH-4102 Binningen(CH)**

(72) Erfinder: **Jaeggi, Knut A., Dr.**
**General Guisan-Strasse 44**
**CH-4054 Basel(CH)**

(72) Erfinder: **Rossi, Alberto, Dr.**
**Bündtenweg 30**
**CH-4104 Oberwil(CH)**

(74) Vertreter: **Zumstein, Fritz sen., Dr. et al,**
**Bräuhausstrasse 4**
**D-8000 München 2(DE)**

(54) **Neue Ammoniumsalze, Verfahren zu ihrer Herstellung, diese enthaltende pharmazeutische Präparate und ihre Verwendung.**

(57) Ammoniumsalze substituierter 3,4-Dihydro-2H-1,2-benzothiazin-1,1-dioxide der Formel I

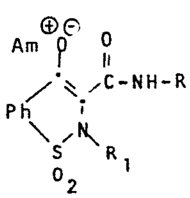

(I),

worin Ph einen gegebenenfalls substituierten 1,2-Phenylenrest bedeutet, R einen gegebenenfalls substituierten Heteroarylrest darstellt, $R_1$ Wasserstoff oder einen gegebenenfalls substituierten Kohlenwasserstoffrest aliphatischen Charakters bedeutet und $Am^{\oplus}$ das von einem pharmazeutisch verwendbaren Dieniederalkyl-, Di-(hydroxyniederalkyl)-oder Tri-(hydroxyniederalkyl)-amin abgeleitete Ammonium-Kation darstellt, haben gegenüber bekannten Salzen von Verbindungen der Formel II

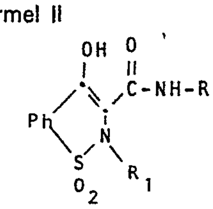

(II)

gewichtige Vorteile. Die neuen Salze werden hergestellt, indem man eine Säure der Formel I oder ein Salz davon mit dem Amin Am-H (III) oder einem Salz davon umsetzt.

Die neuen Ammoniumsalze der Formel I können Verwendung finden als Arzneimittelwirkstoffe in antiinflammatorischen Arzneimitteln.

EP 0 082 217 A1

0082217



CIBA-GEIGY AG
Basel (Schweiz)

4-13662/11566

# Neue Ammoniumsalze, Verfahren zu ihrer Herstellung, diese enthaltende pharmazeutische Präparate und ihre Verwendung

Die Erfindung betrifft neue Ammoniumsalze substituierter 3,4-Dihydro-2H-1,2-benzothiazin-1,1-dioxide der Formel I

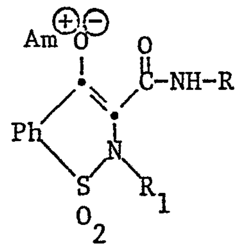

(I),

worin Ph einen gegebenenfalls substituierten 1,2-Phenylenrest bedeutet, R einen gegebenenfalls substituierten Heteroarylrest darstellt, $R_1$ Wassersroff oder einen gegebenenfalls substituierten Kohlenwasserstoffrest aliphatischen Charakters bedeutet und $Am^{\oplus}$ das von einem pharmazeutisch verwendbaren Diniederalkyl-, Di-(hydroxyniederalkyl)- oder Tri-(hydroxyniederalkyl)-amin abgeleitete Ammonium-Kation darstellt, sowie Verfahren zu ihrer Herstellung, diese enthaltende pharmazeutische Präparate und ihre Verwendung.

Als Substituenten eines 1,2-Phenylenrestes Ph kommen beispielsweise Niederalkyl, Niederalkoxy, Niederalkanoyl, Halogen, Trifluormethyl und/oder Nitro in Betracht.

Der gegebenenfalls substituierte Heteroarylrest kann über ein beliebiges Kohlenstoffatom (C-Atom) gebunden sein und ist beispielsweise ein gegebenenfalls substituierter, als Heteroatom(e) ein Sauerstoffatom, ein Sauerstoff- oder Schwefelatom sowie ein Stickstoffatom

oder ein Schwefelatom sowie zwei Stickstoffatome aufweisender 5-gliedriger oder als Heteroatom(e) ein, zwei oder drei Stickstoffatome aufweisender 6-gliedriger monocyclischer oder als Heteroatom ein Sauerstoffatom aufweisender 10-gliedriger, aus zwei 6 Ringen aufgebauter bicyclischer Heteroarylrest.

Als Substituenten dieser Heteroarylreste kommen beispielsweise aliphatische Kohlenwasserstoffreste, gegebenenfalls veräthertes oder verestertes Hydroxy, Acyl, gegebenenfalls substituiertes Amino und Trifluormethyl in Betracht. Ein als Heteroatom Sauerstoff aufweisender 10-gliedriger bicyclischer Heteroarylrest trägt zudem an dem das Sauerstoffatom aufweisenden Ring stets eine Oxogruppe.

Letzterer stellt somit einen Benzopyronrest dar und ist beispielsweise ein gegebenenfalls substituierter, in 4-, 6- oder vor allem 3- oder 7-Stellung gebundener 2-Oxo-2H-1-benzopyranylrest oder in 3- oder in zweiter Linie 6- oder 7-Stellung gebundener 4-Oxo-4H-1-benzopyranylrest.

Als monocyclische Ringsysteme der vorstehend definierten Art sind beispielsweise gegebenenfalls wie angegeben substituiertes Furyl, Isoxazolyl, Thiazolyl, Isothiazolyl, Thiadiazolyl, Pyridyl, Pyrazinyl, Pyrimidyl sowie Pyridazinyl zu nennen.

Aliphatische Kohlenwasserstoffreste sind beispielsweise Niederalkyl oder an zwei benachbarte Kohlenstoffatome, insbesondere i 5,6-, 6,7- oder 7,8-Stellung oder in 3,4-Stellung eines 2-Oxo-2H-1-benzopyran-7-ylrestes, gebundenes 3- oder 4-gliedriges Niederalkylen.

Veräthertes Hydroxy ist beispielsweise Niederalkoxy oder an zwei benachbarte Kohlenstoffatome eines Benzopyronrestes, z.B. in 6,7-Stellung,gebundenes 3- oder 4-gliedriges Niederalkylendioxy.

Verestertes Hydroxy ist beispielsweise mit einer Mineralsäure oder einer Carbonsäure verestertes Hydróxy, wie Halogen, Niederalkanoyloxy oder gegebenenfalls durch Niederalkyl, Niederalkoxy, Halogen, Trifluormethyl und/oder Nitro substituiertes Benzoyloxy.

Substituierte Aminogruppen sind beispielsweise durch aliphatische Kohlenwasserstoffreste, z.B. Niederalkyl, oder Acyl, z.B. Niederalkanoyl, substituierte Aminogruppen, wie N-Mono- oder N,N-Diniederalkylenamino oder Niederalkanoylamino.

Acyl ist beispielsweise Niederalkanoyl oder gegebenenfalls durch Niederalkyl, Niederalkoxy, Halogen und/oder Nitro substituiertes Benzoyl.

Ein gegebenenfalls substituierter Kohlenwasserstoffrest $R_1$ aliphatischen Charakters ist beispielsweise gegebenenfalls substituiertes Niederalkyl, wie Niederalkyl, Hydroxyniederalkyl oder Niederalkoxyniederalkyl, Niederalkenyl oder gegebenenfalls durch Niederalkyl, Niederalkoxy, Halogen, Trifluormethyl und/oder Nitro substituiertes Phenylniederalkyl.

Bevorzugte Reste R sind: unsubstituiertes oder in 2-Stellung durch Niederalkyl, in 5-, 6-, 7- und/oder 8-Stellung durch Niederalkyl, Niederalkoxy und/oder Halogen oder in 5,6-, 6,7- oder 7,8-Stellung durch Niederalkylen oder Niederalkylendioxy substituiertes 4-Oxo-4H-1-benzopyran-3-yl, 2-Furyl, unsubstituiertes oder in 3-, 4-, 5- oder 6-Stellung oder 4- und 5-Stellung durch Niederalkyl oder in 5-Stellung durch Halogen oder Hydroxy substituiertes 2-Pyridyl, unsubstituiertes oder in 4- und/oder 5-Stellung durch Niederalkyl substituiertes 3-Isoxazolyl bzw. unsubstituiertes oder in 3- und/oder 4-Stellung durch Niederalkyl substituiertes 5-Isoxazolyl, unsubstituiertes oder in 4- und/oder 5-Stellung durch Niederalkyl substituiertes 2-Thiazolyl, 3-Isothiazolyl, unsubstituiertes oder in 5-Stellung durch Niederalkyl substituiertes 1,3,4-Thiadiazolyl-(2),

2-Pyrazinyl, unsubstituiertes oder in 4- und/oder 5-Stellung durch Niederalkyl substituiertes 2-Pyrimidinyl, 4-Pyrimidinyl sowie unsubstituiertes oder in 5-Stellung durch Niederalkyl oder in 6-Stellung durch Niederalkoxy substituiertes 3-Pyridazinyl.

Vor- und nachstehend haben die allgemeinen Begriffe folgende Bedeutung:
Niedere Reste enthalten beispielsweise bis zu 7, vor allem bis zu 4 Kohlenstoffatome.

Niederalkyl ist beispielsweise Methyl, Aethyl, Propyl, Isopropyl, n-Butyl, Isobutyl, sek.-Butyl oder tert.-Butyl, ferner n-Pentyl, n-Hexyl, Isohexyl oder n-Heptyl.
Niederalkylen ist beispielsweise 1,3-Propylen, 1,4-Butylen oder 1,5-Pentylen.
Phenylniederalkyl ist beispielsweise Benzyl, 1- oder 2-Phenyläthyl oder 3-Phenylpropyl.
Niederalkoxy ist beispielsweise Methoxy, Aethoxy, n-Propyloxy, Isopropyloxy oder n-Butyloxy.
Niederalkoxyniederalkyl ist beispielsweise 2-Methoxyäthyl oder 2-Aethoxyäthyl, während Hydroxyniederalkyl insbesondere 2-Hydroxyäthyl oder 3-Hydroxypropyl bedeutet.
Niederalkylendioxy ist beispielsweise 1,2-Aethylendioxy, 1,3-Propylendioxy oder Methylendioxy.
Niederalkanoyl ist beispielsweise Acetyl, Propionyl, Butyryl, Isobutyryl, Valeroyl oder Pivaloyl.
Niederalkanoyloxy ist beispielsweise Acetoxy, Propionyloxy, Butyryloxy, Isobutyryloxy, Valeroyloxy oder Pivaloyloxy.
Niederalkanoylamino ist beispielsweise Acetamino, Propionylamino oder Butyrylamino.
N-Mono- oder N,N-Diniederalkylamino ist beispielsweise N-Methyl-, N,N-Dimethyl- oder N,N-Diäthylamino.
Diniederalkylammonium $Am^{\oplus}$ ist beispielsweise Di-$C_2$-$C_4$-alkylammonium, wie Diäthylammonium.

Di-(hydroxyniederalkyl)- bzw. Tri-(hydroxyniederalkyl)-ammonium $Am^{\oplus}$ ist beispielsweise Di-(2-hydroxy-$C_2$-$C_4$-alkyl)- bzw. Tri-(2-hydroxy-$C_2$-$C_4$-alkyl)-ammonium, wie Diäthanolammonium oder vor allem Triäthanolammonium.

Die den neuen Ammoniumsalzen zugrundeliegenden freien Säuren der Formel

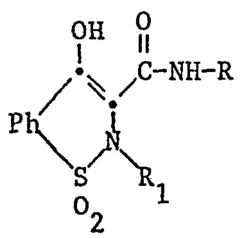

(II)

sind bekannt, auch einige Salze derselben mit anorganischen Basen. So betrifft die US-Patentschrift Nr. 4,213,980 Säuren der Formel II, worin R einen gegebenenfalls substituierten Benzopyronrest darstellt, und deren "Salze von Metallen der Gruppen I und II des periodischen Systems, Ammoniumsalze sowie Salze mit organischen Basen, wie geeigneten Aminen, z.B. Aethylamin, Triäthylamin, Diäthylaminoäthanol, Aethylendiamin, Benzylamin, Prokain, Pyrrolidin, Piperidin, Morpholin, 1-Aethylpiperidin und 2-Piperidinoäthanol". Spezifisch beschrieben sind indes lediglich das Natrium-, Zink- und Kupfersalz von 4-Hydroxy-2-methyl-N-(4-oxo-4H-1-benzopyran-3-yl)-2H-1,2-benzothiazin-3-carboxamid-1,1-dioxid. Ferner betrifft die US-Patentschrift Nr. 3,591,584 Verbindungen der Formel II, worin R eine Vielzahl von Heteroarylresten bedeutet und "pharmazeutisch verwendbare Salze" derselben, wobei "Natrium-, Kalium-, Calcium-, Magnesiumsalze und dergl." beispielhaft erwähnt werden, unter Hervorhebung der Natriumsalze. Auch ergibt sich aus den US-Patentschriften Nr. 3,822,258 und 3,868,367, dass Verbindungen der Formel II, worin Ph unsubstituiertes 1,2-Phenylen ist, R einen 4-$R_3$-5-$R_2$-Isoxazolyl-(3)-rest (US 3,822,258) bzw. 3-$R_3$-4-$R_2$-Isoxazolyl-(5)-rest (US 3,868,367) bedeutet und $R_1$, $R_2$ und $R_3$ Wasserstoff oder Alkyl darstellen, "Salze von Alkalimetallen, Erdalkalimetallen und Aminen" bilden, wobei als salzbildende Amine "Pyrrolidin und dergleichen" (US 3,822,258) bzw. Triäthanolamin

(US 3,868,367) erwähnt werden. Beschrieben ist jedoch lediglich das Natriumsalz von 4-Hydroxy-2-methyl-N-(5-methyl-3-isoxazolyl)-2H-1,2-benzothiazin-carboxamid-1,1-dioxid. Von der pauschalen Erwähnung von Triäthanolammoniumsalzen von 5-Isoxazolylverbindungen,von denen indes auch keine einzige beschrieben ist,fehlt abgesehen davon in der genannten und und weiteren Druckschriften jeder Hinweis auf ein Diniederalkyl-, Dihydroxyniederalkyl)- oder Tri-(hydroxyniederalkyl)-amine als salzbildende Basen. Es fehlt im Stand der Technik auch jedweder Anhaltspunkt,dass diese oder Salze von Säuren der Formel I mit irgendwelchen organischen Aminen den bekannten Metallsalzen gegenüber Vorteile aufweisen oder Nachteile derselben vermeiden könnten. Die Ammoniumsalze der Formel I sind dementsprechend als derzeit noch neu anzusehen. Sie weisen auch nicht zu erwartende Vorteile auf. Die Einsatzmöglichkeiten der Verbindungen der Formel II und ihrer derzeit bekannten Salze als antiinflammatorische Arzneimittelwirkstoffe werden nämlich durch ihre verlangsamte Resoprtion im Organismus nicht unerheblich eingeschränkt. Dieser Nachteil der bekannten Verbindungen ist nach derzeitigem Erkenntnisstand auf unzureichende Löslichkeit der freien Verbindungen der Formel II im sauren Milieu des Magens zurückzuführen. Die Resorption lässt sich auch durch Verabreichung einer bekannten Salzform nicht wesentlich beschleunigen, da diese bereits im Magen durch die Magensäure in die freie Säure überführt werden. Davon abgesehen, sind einige der bekannten Salze stark hygroskopisch, so dass sie nicht oder nur mit unvertretbarem Aufwand in eine stabile pharmazeutische Darreichungsform gebracht werden können.

Der anmeldungsgemässen Erfindung liegt die überraschende, nach Art und Ausmass nicht voraussehbare Feststellung zugrunde, dass die Ammoniumsalze der Formel I wesentlich schneller und besser resorbiert werden als die bekannten Salze. Dies kann beispielsweise anhand der für einen bestimmten Therapieerfolg erforderlichen peroralen Dosen dokumentiert werden. So wurde beispielsweise festgestellt, dass das

Triäthanolammoniumsalz des 4-Hydroxy-2-methyl-N-(4-oxo-4H-1-benzopyran-3-yl)-2H-1,2-benzothiazin-3-carboxamid-1,1-dioxides im Kaolin-Pfotenoedem der Ratte sowie im Phenyl-p-benzochinon-Writhingsyndrom der Maus bei wesentlich geringeren Dosen als das bekannte Natriumsalz-Monohydrat therapeutisch wirksam ist. Im Gegensatz zu letztgenannten ist es auch nicht hygroskopisch.

Die neuen Ammoniumsalze sind dementsprechend vorzüglich geeignet als Antiinflammatorika und/oder Analgetika bei entzündlichen Vorgängen verschiedener Aethiologie, insbesondere des rheumatischen Formenkreises, vor allem zur Behandlung akuter Schübe.

Die Erfindung betrifft vor allem Verbindungen der Formel I, worin Ph einen gegebenenfalls durch Niederalkyl, Niederalkoxy, Niederalkanoyl, Halogen, Nitro und/oder Trifluormethyl substituierten 1,2-Phenylenrest bedeutet, R ein gegebenenfalls durch Niederalkyl, Niederalkoxy, Halogen, Niederalkanoyloxy, Hydroxy, Benzoyloxyreste, gegebenenfalls durch Niederalkyl oder Niederalkanoyl substituiertes Amino substituierter, als Heteroatom(e) ein Sauerstoffatom, ein Sauerstoff- oder Schwefelatom sowie ein Stickstoffatom oder ein Schwefelatom sowie zwei Stickstoffatome aufweisender 5-gliedriger oder als Heteroatom(e) ein, zwei oder drei Stickstoffatome aufweisender 6-gliedriger monocyclischer Heteroarylrest oder ein gegebenenfalls durch Niederalkyl, Niederalkoxy, Halogen, Niederalkanoyloxy, Hydroxy, Benzoyloxyreste, gegebenenfalls durch Niederalkyl oder Niederalkanoyl substituiertes Amino und/oder an zwei benachbarten Kohlenstoffatomen im carbocyclischen Ring durch Niederalkylen oder Niederalkylendioxy substituierter, als Heteroatom ein Sauerstoffatom aufweisender 10-gliedriger, aus zwei 6-Ringen aufgebauter, im sauerstoffhaltigen Ring Oxo aufweisender bicyclischer Heteroarylrest ist und $R_1$ Wasserstoff, Niederalkyl, Niederalkenyl oder einen Phenylniederalkylrest darstellt, wobei in Benzoyl- und Phenylniederalkylgruppen das Phenyl durch Niederalkyl, Niederalkoxy, Halogen, Trifluormethyl und/oder Nitro substituiert sein kann.

Die Erfindung betrifft besonders Verbindungen der Formel I, worin Ph einen unsubstituierten oder durch Niederalkyl mit bis zu 4 Kohlenstoffatomen, wie Methyl, Niederalkoxy mit bis zu 4 Kohlenstoffatomen, wie Methoxy oder Aethoxy, Halogen bis Atomnummer 35, wie Chlor, Nitro oder Trifluormethyl substituierten 1,2-Phenylenrest bedeutet, R Furyl, Isoxazolyl, Thiazolyl, Isothiazolyl, Thiadiazolyl, Pyridyl, Pyrazinyl, Pyrimidinyl, Pyridazinyl oder 4-Oxo-4H- bzw. 2-Oxo-2H-1-benzopyranyl bedeutet, welches unsubstituiert oder durch Niederalkyl mit bis zu 4 Kohlenstoffatomen, wie Methyl, Niederalkoxy mit bis zu 4 Kohlenstoffatomen, wie Methoxy, Halogen bis Atomnummer 35, wie Chlor, Niederalkanoyloxy mit bis zu 4 Kohlenstoffatomen, wie Acetoxy, Hydroxy, Amino, N-Mono- oder N,N-Diniederalkylamino mit bis zu 4 Kohlenstoffatomen im Alkylteil, wie Dimethylamino, Niederalkanoylamino mit bis zu 4 Kohlenstoffatomen, wie Acetylamino, und/oder, im Falle von 4-Oxo-4H- bzw. 2-Oxo-2H-1-benzopyranyl, an zwei benachbarten Kohlenstoffatomen durch Niederalkylen mit bis zu 4 Kohlenstoffatomen, wie 1,3-Propylen oder 1,4-Butylen, oder Niederalkylendioxy mit bis zu 4 Kohlenstoffatomen, wie Methylendioxy oder 1,2-Aethylendioxy, substituiert sein kann, und $R_1$ Wasserstoff oder Niederalkyl mit bis zu 4 Kohlenstoffatomen, wie Methyl oder Aethyl bedeutet.

Die Erfindung betrifft ganz besonders Verbindungen der Formel I, worin Ph gegebenenfalls durch Niederalkyl mit bis zu 4 Kohlenstoffatomen, z.B. Methyl, Niederalkoxy mit bis zu 4 Kohlenstoffatomen, z.B. Methoxy, Halogen bis Atomnummer 35, z.B. Chlor, Trifluormethyl oder Nitro monosubstituiertes 1,2-Phenylen bedeutet, $R_1$ Wasserstoff oder vor allem Niederalkyl mit bis zu 4 Kohlenstoffatomen, z.B. Methyl oder Aethyl, bedeutet, und R Furyl, wie 2-Furyl, gegebenenfalls in 3- und/ oder 4- bzw. 4- und/oder 5-Stellung durch Niederalkyl mit bis und mit 4 C-Atomen, wie Methyl, mono- oder disubstituiertes Isoxazolyl, wie 5-Methyl-3-isoxazolyl oder 3-Methyl-5-isoxazolyl, gegebenenfalls in 4- und/oder 5-Stellung durch Niederalkyl mit bis und mit 4 C-Atomen,

wie Methyl, substituiertes Thiazolyl, wie 2-Thiazolyl, 4-Methyl-2-thiazolyl oder 4,5-Dimethyl-2-thiazolyl, Isothiazolyl, wie 3-Isothiazolyl, gegebenenfalls in 5-Stellung durch Niederalkyl mit bis und mit 4 C-Atomen, wie Methyl, substituiertes Thiadiazolyl, wie 1,3,4-Thiadiazolyl-(2) oder 5-Methyl-2-thiadiazolyl, gegebenenfalls in 3-, 4-, 5-, 6- oder 4- und 5-Stellung durch Niederalkyl mit bis und mit 4 C-Atomen, wie Methyl oder in 5-Stellung durch Halogen der Atomnummer bis und mit 35, wie Chlor oder Brom, oder Hydroxy substituiertes Pyridyl, wie 2-Pyridyl, 3-, 4-, 5- oder 6-Methyl-2-pyridyl, 5-Chlor- oder 5-Brom-2-pyridyl oder 3-Hydroxy-2-pyridyl, Pyrazinyl, wie 2-Pyrazinyl, gegebenenfalls in 5-Stellung durch Niederalkyl oder in 6-Stellung durch jeweils Niederalkoxy mit bis und mit 4 C-Atomen, wie Methyl oder Methoxy, substituiertes Pyridazinyl, wie 5-Methyl-3-pyridazinyl oder 6-Methoxy-3-pyridazinyl, gegebenenfalls in 4- und/oder 5-Stellung durch Niederalkyl mit bis und mit 4 C-Atomen, wie Methyl, substituiertes Pyrimidinyl, wie 2- oder 4-Pyrimidinyl oder 4,5-Dimethyl-2-pyrimidinyl, gegebenenfalls in 3- und/oder 4-Stellung durch Niederalkyl und/oder Niederalkoxy mit bis und mit 4 C-Atomen, wie Niederalkyl und/oder Niederalkoxy, 3- oder 4-gliedriges Niederalkylen bzw. Niederalkylendioxy, z.B. 1,3-Propylen, 1,4-Butylen, Methylendioxy oder 1,2-Aethylendioxy, oder in 4-Stellung durch Hydroxy substituiertes 2-Oxo-2H-1-benzopyran-7-yl oder gegebenenfalls im Pyranteil durch Niederalkyl mit bis und mit 4 C-Atomen, wie Methyl, und/oder im Benzoteil durch Niederalkyl mit bis und mit 4 C-Atomen, wie Methyl, Niederalkoxy mit bis und mit 4 C-Atomen, wie Methoxy , und/oder Halogen der Atomnummer bis und mit 35, wie Chlor, oder an zwei benachbarten C-Atomen durch 3- oder 4-gliedriges Niederalkylen bzw. Niederalkylendioxy, wie 1,3-Propylen oder Aethylendioxy, substituiertes 4-Oxo-4H-1-benzopyran-3-yl bedeutet.

Die Erfindung betrifft vor allem Verbindungen der Formel I, worin $R_1$ Niederalkyl mit bis zu 4 Kohlenstoffatomen, wie Methyl, bedeutet, Ph unsubstituiertes 1,2-Phenylen bedeutet, und R entweder im Benzoteil

gegebenenfalls durch Niederalkyl mit bis zu 4 Kohlenstoffatomen, wie Methyl, Niederalkoxy mit bis und mit 4 C-Atomen, wie Methoxy, und/ oder Halogen der Atomnummer bis und mit 35, wie Chlor, mono- oder disubstituiertes oder an zwei benachbarte C-Atome, vorzugsweise in 6,7-Stellung gebundenes, 3- bis 4-gliedriges Niederalkylen mit bis zu 4 Kohlenstoffatomen, wie 1,3-Propylen, disubstituiertes 4-Oxo-4H-1-benzopyran-3-yl bedeutet, oder andererseits 2-Thiazolyl, 5-Methyl-3-isoxazolyl oder 2-Pyridyl darstellt.

Die Erfindung betrifft jeweils vorzugsweise diejenigen der vorstehend definierten Verbindungen, worin $Am^{\oplus}$ Di-$C_2$-$C_4$-alkylammonium, Di-(2-hydroxy-$C_2$-$C_4$-alkyl)-ammonium oder Tri-(2-hydroxy-$C_2$-$C_4$-alkyl)-ammonium, vorzugsweise Diäthylammonium, Diäthanolammonium oder ganz besonders Triäthanolammonium bedeutet.

Die Erfindung betrifft in allererster Linie Verbindungen der Formel I, worin Ph unsubstituiertes 1,2-Phenylen ist, $R_2$ Methyl bedeutet und R 4-Oxo-4H-1-benzopyran-3-yl, 2-Thiazolyl, 5-Methyl-3-isoxazolyl oder 2-Pyridyl darstellt und $Am^{\oplus}$ für Triäthanolammonium steht.

Die Erfindung betrifft namentlich die in den Beispielen genannten Verbindungen der Formel I.

Die neuen Verbindungen der Formel I können nach an sich bekannten Methoden hergestellt werden, beispielsweise indem man eine Verbindung der Formel II

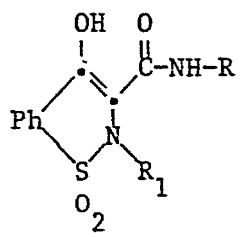

(II)

oder ein Salz davon mit einem Diniederalkyl-, D-(hydroxyniederalkyl)- oder Tri-(hydroxyniederalkyl)-amin der Formel (III) oder einem Salz

davon umsetzt.

Verfahrensgemäss verwendbare Salze von Verbindungen der Formel II sind insbesondere Salze mit Basen, die aus dem Reaktionsgemisch entfernt werden können, beispielsweise flüchtiger oder schwächer sind als das Amin der Formel III oder mit der betreffenden Verbindung der Formel II leichter lösliche Salze bilden als dieses, beispielsweise Ammoniumsalze mit Ammoniak, ebenso Metallsalze, die bei der Umsetzung mit einem geeigneten Salz des Amins der Formel III schwerlösliche Salze bilden, wie Calciumsalze.

Verfahrensgemäss verwendbare Salze von Aminen der Formel III sind beispielsweise Salze derselben mit Säuren, die aus dem Reaktionsgemisch entfernt werden können, beispielsweise entsprechende Ammoniumsalze von flüchtigen Säuren oder von Säuren, die schwächer sauer sind als die entsprechenden Salze der Formel I, wie Salze von Aminen der Formel III mit anorganischen Säuren,z.B. Hydrohalogenide oder Sulfate, oder Salze mit flüchtigen Carbonsäuren oder Mono- bzw. Dithiocarbonsäuren, z.B. Salze mit Niederalkansäuren, Kohlensäure oder Dithiokohlensäure bzw. deren Halbester oder Halbamide, u.a. entsprechende Triäthanol-Acetate, Carbonate bzw. Hydrogencarbonate, Carbaminate bzw. Dithiocarbaminate oder Niederalkoxyformiate, ebenso Ammoniumhydroxide der Formel $[AmH_2]^{\oplus}OH^{\ominus}$.

Die Umsetzung erfolgt in üblicher Weise, vorteilhaft in einem Lösungs- oder Verdünnungsmittel, erforderlichenfalls unter Entfernung, wie destillativer bzw. azeotrop-destillativer Entfernung flüchtiger Reaktionsteilnehmer, wie Wasser, Ammoniak, Kohlendioxyd, Schwefelkohlenstoff oder einer gebildeten Halogenwasserstoffsäure oder Niederalkansäure, oder unter Ausfällung schwerlöslicher Salze, z.B. von Calciumsulfat und/oder unter Erwärmen.

In einer bevorzugten Ausführungsform des Verfahrens beispielsweise

setzt man die betreffende Säure der Formel II in einem organischen Lösungsmittel direkt mit dem Amin der Formel III um.

In einer weiteren bevorzugten Ausführungsform setzt man die Säure der Formel II mit dem Carbonat, Hydrogencarbonat oder Carbaminat des Amins der Formel III unter Entfernung des gebildeten Kohlendioxids und gegebenenfalls Ammoniaks um.

In einer weiteren bevorzugten Ausführungsform setzt man das von Ammoniak abgeleitete Ammoniumsalz der betreffenden Säure der Formel II mit dem Amin um, vorteilhaft unter destillativer Entfernung des gebildeten Ammoniaks oder Bindung desselben an einen sauren Ionenaustauscher.

In einer weiteren bevorzugten Ausführungsform geht man von einem organischen Salz, z.B. dem Natriumsalz, der Säure der Formel II aus und setzt dieses in einem Lösungsmittel, in dem das gebildete anorganische Salz, z.B. Natriumchlorid, schwerer löslich ist als das gebildete Salz der Formel I, mit einem von Salzen der Formel I verschiedenen Salz des Imins, z.B. einem Hydrochlorid, um.

Zweckmässig verwendet man für die Durchführung der erfindungsgemässen Reaktionen solche Ausgangsstoffe, die zu den eingangs besonders erwähnten Gruppen von Endstoffen und vor allem zu den speziell beschriebenen oder hervorgehobenen Endstoffen führen.

Bei den erfindungsgemässen pharmazeutischen Präparaten, welche pharmazeutisch verwendbare Salze der Formel I enthalten, handelt es sich um solche zur enteralen, wie oralen oder rektalen, sowie parenteralen Verabreichung an Warmblüter, welche den pharmakologischen Wirkstoff allein oder zusammen mit einem pharmazeutisch anwendbaren Trägermaterial enthalten.

Die neuen pharmazeutischen Präparate enthalten von etwa 10% bis etwa 95%, vorzugsweise von etwa 20% bis etwa 90%, des Wirkstoffes. Erfindungsgemässe pharmazeutische Präparate in Dosiseinheitsform, wie Dragées, Tabletten, Kapseln, Suppositorien oder Ampullen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister z.B. Mais-, Weizen-, Reis- oder Kartoffelstärke, Kleister, Gelatine, Traganth, Methylcellulose, Hydroxypropyl-methylcellulose, Natriumcarboxymethylcellulose, und/oder Polyvinylpyrrolidon, und/oder, wenn erwünscht, Sprengmittel, wie die obengenannten Stärlen, ferner Carboxymethylstärke, quervernetztes Polyvinylpyrrolidon, Agar, Alginsäure oder ein Salz davon, wie Natriumalginat, Hilfsmittel sind in erster Linie Fliessregler- und Schmiermittel, z.B. Kieselsäure, Talk, Stearinsäure oder Salze davon, wie Magnesium- oder Calciumstearat, und/oder Polyäthylenglykol. Dragéee-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten. Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifzierung oder zur Kennzeichnung verschiedener Wirkstoffe beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-Carboxymethylcellulose, Sorbit und/oder Dextran und gegebenenfalls auch Stabilisatoren enthalten.

Die erfindungsgemässen pharmazeutisch verwendbaren Salze der Formel I können als pharmakologisch wirksame Stoffe, insbesondere als Antiphlogistika, sowie als Analgetika, vorzugsweise in Form von pharmazeutischen Präparaten, verwendet werden. Die Dosierung des Wirkstoffes hängt vom Warmblüter-Spezies, dem Körpergewicht und Alter und vom individuellen Zustand, sowie von der Applikationsweise ab. Durchschnittlich

und Alter und vom individuellen Zustand, sowie von der Applikationsweise ab. Durchschnittlich wird einem Warmblüter von etwa 70 kg Körpergewicht eine Tagesdosis von etwa 50 bis etwa 200 mg, vorzugsweise von etwa 75 bis etwa 150 mg Wirkstoff verabreicht.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

Beispiel 1: 40 g 4-Hydroxy-2-methyl-N-(4-oxo-4H-1-benzopyran-3-yl)-2H-1,2-benzothiazin-3-carboxamid-1,1-dioxid werden in 1000 ml Dimethylformamid suspendiert. Dann fügt man 30 g Triäthanolamin hinzu und erwärmt eine Stunde unter Rühren auf 100°. Dann gibt man weitere 30 g Triäthanolamin hinzu und dampft unter vermindertem Druck bei 60° zur Trockne ein. Der dickflüssige, gelbe Rückstand wird unter gelindem Erwärmen in 600 ml Aceton gelöst. Man engt etwas ein, lässt auf Raumtemperatur abkühlen, filtriert das ausgefallene gelbe Kristallisat ab und trocknet unter vermindertem Druck bei 80° bis zur Gewichtskonstanz. Man erhält das Triäthanolammoniumsalz des 4-Hydroxy-2-methyl-N-(4-oxo-4H-1-benzopyran-3-yl)-2H-1,2-benzothiazin-3-carboxamid-1,1-dioxides vom Smp. 220° (Zers.).

In analoger Weise erhält man ausgehend von je 0,2 Mol
4-Hydroxy-2-methyl-N-(4,6,7,8-tetrahydro-4-oxo-cyclopenta[g]-1-benzopyran-3- yl)-2H-1,2-benzothiazin-3-carboxamid-1,1-dioxid,
4-Hydroxy-2-methyl-N-(4-oxo-6-methyl-4H-1-benzopyran-3-yl)-2H-1,2-benzothiazin-3-carboxamid-1,1-dioxid,
4-Hydroxy-2-methyl-N-(4-oxo-8-methyl-4H-1-benzopyran-3-yl)-2H-1,2-benzothiazin-3-carboxamid-1,1-dioxid,
4-Hydroxy-2-methyl-N-(2-oxo-4-hydroxy-6-methyl-2H-1-benzopyran-3-yl)-2H-1,2-benzothiazin-3-carboxamid-1,1-dioxid,
4-Hydroxy-2-methyl-N-(4-oxo-6-chlor-8-methyl-4H-1-benzopyran-3-yl)-2H-1,2-benzothiazin-3-carboxamid-1,1-dioxid,

4-Hydroxy-2-methyl-N-(4-oxo-6,7-dimethyl-4H-1-benzopyran-3-yl)-2H-1,2-benzothiazin-3-carboxamid-1,1-dioxid,
4-Hydroxy-2-methyl-N-(4-oxo-6-methoxy-4H-1-benzopyran-3-yl)-2H-1,2-benzothiazin-3-carboxamid-1,1-dioxid,
4-Hydroxy-2-methyl-N-(4-oxo-5,7-dimethyl-4H-1-benzopyran-3-yl)-2H-1,2-benzothiazin-3-carboxamid-1,1-dioxid,
4-Hydroxy-2-methyl-N-(2-oxo-4-hydroxy-6-chlor-2H-1-benzopyran-3-yl)-2H-1,2-benzothiazin-3-carboxamid-1,1-dioxid,
4-Hydroxy-2-methyl-N-(2-oxo-4-hydroxy-6,8-dichlor-2H-1-benzopyran-3-yl)-2H-1,2-benzothiazin-3-carboxamid-1,1-dioxid,
4-Hydroxy-2-methyl-N-(2-oxo-4-hydroxy-4,6,7,8-tetrahydro-cyclopenta-[g]2H-1-benzopyran-3-yl)-2H-1,2-benzothiazin-3-carboxamid-1,1-dioxid,
4-Hydroxy-2-methyl-N-(2-oxo-4-hydroxy-8-methyl-2H-1-benzopyran-3-yl)-2H-1,2-benzothiazin-3-carboxamid-1,1-dioxid,
4-Hydroxy-2-methyl-N-(2-oxo-4-methyl-2H-1-benzopyran-7-yl)-2H-1,2-benzothiazin-3-carboxamid-1,1-dioxid,
4-Hydroxy-2-methyl-N-(6-chlor-7-methyl-4-oxo-4H-1-benzopyran-3-yl)-2H-1,2-benzothiazin-3-carboxamid-1,1-dioxid,
2-Aethyl-N-4-hydroxy-N-(4-oxo-4H-1-benzopyran-3-yl)-2H-1,2-benzothiazin-3-carboxamid-1,1-dioxid,
2-Allyl-4-hydroxy-N-(4-oxo-4H-1-benzopyran-3-yl)-2H-1,2-benzothiazin-3-carboxamid-1,1-dioxid,
4-Hydroxy-2-methyl-N-(2-pyridyl)-2H-1,2-benzothiazin-3-carboxamid-1,1-dioxid,
4-Hydroxy-2-methyl-N-(2-thiazolyl)-2H-1,2-benzothiazin-3-carboxamid-1,1-dioxid,
4-Hydroxy-2-methyl-N-(2-furyl)-2H-1,2-benzothiazin-3-carboxamid-1,1-dioxid,
4-Hydroxy-2-methyl-N-(3-methyl-isoxazol-3-yl)-2H-1,2-benzothiazin-3-carboxamid-1,1-dioxid,
das Triäthanolammoniumsalz des 4-Hydroxy-2-methyl-N-(4,6,7,8-tetrahydro-cyclopenta[g]-1-benzopyran-3-yl)-2H-1,2-benzothiazin-3-carboxamid-1,1-dioxids,

das Triäthanolammoniumsalz des 4-Hydroxy-2-methyl-N-(4-oxo-6-methyl-4H-1-benzopyran-3-yl)-2H-1,2-benzothiazin-3-carboxamid-1,1-dioxids,
das Triäthanolammoniumsalz des 4-Hydroxy-2-methyl-N-(4-oxo-8-methyl-4H-1-benzopyran-3-yl)-2H-1,2-benzothiazin-3-carboxamid-1,1-dioxids,
das Triäthanolammoniumsalz des 4-Hydroxy-2-methyl-N-(2-oxo-4-hydroxy-6-methyl-2H-1-benzopyran-3-yl)-2H-1,2-benzothiazin-3-carboxamid-1,1-dioxids,
das Triäthanolammoniumsalz des 4-Hydroxy-2-methyl-N-(4-oxo-6-chlor-8-methyl-4H-1-benzopyran-3-yl)-2H-1,2-benzothiazin-3-carboxamid-1,1-dioxids,
das Triäthanolammoniumsalz des 4-Hydroxy-2-methyl-N-(4-oxo-6,7-dimethyl-4H-1-benzopyran-3-yl)-2H-1,2-benzothiazin-3-carboxamid-1,1-dioxids,
das Triäthanolammoniumsalz des 4-Hydroxy-2-methyl-N-(4-oxo-6-methoxy-4H-1-benzopyran-3-yl)-2H-1,2-benzothiazin-3-carboxamid-1,1-dioxids,
das Triäthanolammoniumsalz des 4-Hydroxy-2-methyl-N-(4-oxo-5,7-dimethyl-4H-1-benzopyran-3-yl)-2H-1,2-benzothiazin-3-carboxamid-1,1-dioxids,
das Triäthanolammoniumsalz des 4-Hydroxy-2-methyl-N-(2-oxo-4-hydroxy-6-chlor-2H-1-benzopyran-3-yl)-2H-1,2-benzothiazin-3-carboxamid-1,1-dioxids,
das Triäthanolammoniumsalz des 4-Hydroxy-2-methyl-N-(2-oxo-4-hydroxy-6,8-dichlor-2H-1-benzopyran-3-yl)-2H-1,2-benzothiazin-3-carboxamid-1,1-dioxids,
das Triäthanolammoniumsalz des 4-Hydroxy-2-methyl-N-(2-oxo-4-hydroxy-4,6,7,8-tetrahydro-cyclopenta[g]-2H-1-benzopyran-3-yl)-2H-1,2-benzothiazin-3-carboxamid-1,1-dioxids,
das Triäthanolammoniumsalz des 4-Hydroxy-2-methyl-N-(2-oxo-4-hydroxy-8-methyl-2H-1-benzopyran-3-yl)-2H-1,2-benzothiazin-3-carboxamid-1,1-dioxids,
das Triäthanolammoniumsalz des 4-Hydroxy-2-methyl-N-(2-oxo-4-methyl-2H-1-benzopyran-7-yl)-2H-1,2-benzothiazin-3-carboxamid-1,1-dioxids,
das Triäthanolammoniumsalz des 4-Hydroxy-2-methyl-N-(6-chlor-7-methyl-4-oxo-4H-1-benzopyran-3-yl)-2H-1,2-benzothiazin-3-carboxamid-1,1-dioxids,

das Triäthanolammoniumsalz des 2-Aethyl-N-4-hydroxy-N-(4-oxo-4H-1-benzopyran-3-yl)-2H-1,2-benzothiazin-3-carboxamid-1,1-dioxids,
das Triäthanolammoniumsalz des 2-Allyl-4-hydroxy-N-(4-oxo-4H-1-benzopyran-3-yl)-2H-1,2-benzothiazin-3-carboxamid-1,1-dioxids,
das Triäthanolammoniumsalz des 4-Hydroxy-2-methyl-N-(2-pyridyl)-2H-1,2-benzothiazin-3-carboxamid-1,1-dioxids, Smp. 170-172°,
das Triäthanolammoniumsalz des 4-Hydroxy-2-methyl-N-(2-thiazolyl)-2H-1,2-benzothiazin-3-carboxamid-1,1-dioxids,
das Triäthanolammoniumsalz des 4-Hydroxy-2-methyl-N-(2-furyl)-2H-1,2-benzothiazin-3-carboxamid-1,1-dioxids und
das Triäthanolammoniumsalz des 4-Hydroxy-2-methyl-N-(5-methyl-3-isoxazol-yl)-2H-1,2-benzothiazin-3-carboxamid-1,1-dioxids, Smp. 180°C (Zers.).

Beispiel 2: 0,4 g 4-Hydroxy-2-methyl-N-(4-oxo-4H-1-benzopyran-3-yl)-2H-1,2-benzothiazin-3-carboxamid-1,1-dioxid werden in 2 ml Dimethylformamid suspendiert, mit 0,15 g Diäthylamin versetzt und auf 50° erwärmt, bis man eine klare, gelbliche Lösung erhält. Man lässt abkühlen und fällt mit 10 ml Aceton aus. Die ausgefallenen Kristalle werden abgesaugt, mit Aceton gewaschen und getrocknet. Man erhält analysenreines Diäthylammoniumsalz von 4-Hydroxy-2-methyl-N-(4-oxo-4H-1-benzopyran-3-yl)-2H-1,2-benzothiazin-3-carboxamid-1,1-dioxid, welches beim Erhitzen auf 275-277° schmilzt.

In analoger Weise erhält man durch Umsetzung von
2,00 g 4-Hydroxy-2-methyl-N-(4-oxo-4H-1-benzopyran-3-yl)-2H-1,2-benzothiazin-3-carboxamid-1,1-dioxid,
2,20 g 4-Hydroxy-2-methyl-N-(4,6,7,8-tetrahydro-4-oxo-cyclopenta[g]-1-benzopyran-3-yl)-2H-1,2-benzothiazin-3-carboxamid-1,1-dioxid,
2,13 g 4-Hydroxy-2-methyl-N-(6,7-dimethyl-4-oxo-4H-1-benzopyran-3-yl)-2H-1,2-benzothiazin-3-carboxamid-1,1-dioxid,
2,14 g 4-Hydroxy-2-methyl-N-(6-methoxy-4-oxo-4H-1-benzopyran-3-yl)-2H-1,2-benzothiazin-3-carboxamid-1,1-dioxid,

2,13 g 4-Hydroxy-2-methyl-N-(5,7-dimethyl-4-oxo-4H-1-benzopyran-3-yl)-2H-1,2-benzothiazin-3-carboxamid-1,1-dioxid und
2,06 g (4-Hydroxy-2-methyl-N-(8-methyl-4-oxo-4H-1-benzopyran-3-yl)-2H-1,2-benzothiazin-3-carboxamid-1,1-dioxid,
1,68 g 4-Hydroxy-2-methyl-N-(2-thiazolyl)-2H-1,2-benzothiazin-3-carboxamid-1,1-dioxid,
1,61 g 4-Hydroxy-2-methyl-N-(2-pyridyl)-2H-1,2-benzothiazin-3-carboxamid-1,1-dioxid bzw.
1,67 g 4-Hydroxy-2-methyl-N-(5-methyl-3-isoxazolyl)-2H-1,2-benzothiazin-3-carboxamid-1,1-dioxid,
mit jeweils 0,87 g Diäthylamin
das Diäthylammoniumsalz von 4-Hydroxy-2-methyl-N-(4-oxo-4H-1-benzopyran-3-yl)-2H-1,2-benzothiazin-3-carboxamid-1,1-dioxid,
das Diäthylammoniumsalz von 4-Hydroxy-2-methyl-N-(4,6,7,8-tetrahydro-4-oxo-cyclopenta[g]-1-benzopyran-3-yl)-2H-1,2-benzothiazin-3-carboxamid-1,1-dioxid,
das Diäthylammoniumsalz von 4-Hydroxy-2-methyl-N-(6,7-dimethyl-4-oxo-4H-1-benzopyran-3-yl)-2H-1,2-benzothiazin-3-carboxamid-1,1-dioxid,
das Diäthylammoniumsalz von 4-Hydroxy-2-methyl-N-(6-methoxy-4-oxo-4H-1-benzopyran-3-yl)-2H-1,2-benzothiazin-3-carboxamid-1,1-dioxid,
das Diäthylammoniumsalz von 4-Hydroxy-2-methyl-N-(5,7-dimethyl-4-oxo-4H-1-benzopyran-3-yl)-2H-1,2-benzothiazin-3-carboxamid-1,1-dioxid und
das Diäthylammoniumsalz von 4-Hydroxy-2-methyl-N-(8-methyl-4-oxo-4H-1-benzopyran-3-yl)-2H-1,2-benzothiazin-3-carboxamid-1,1-dioxid,
das Diäthylammoniumsalz von 4-Hydroxy-2-methyl-N-(2-thiazolyl)-2H-1,2-benzothiazin-3-carboxamid-1,1-dioxid,
das Diäthylammoniumsalz von 4-Hydroxy-2-methyl-N-(2-pyridyl)-2H-1,2-benzothiazin-1,1-dioxid,
das Diäthylammoniumsalz von 4-Hydroxy-2-methyl-N-(5-methyl-3-isoxazolyl)-2H-1,2-benzothiazin-3-carboxamid-1,1-dioxid.

Beispiel 3: In analoger Weise wie im Beispiel 1 beschrieben erhält man durch Umsetzung von

19,9 g 4-Hydroxy-2-methyl-N-(4-oxo-4H-1-benzopyran-3-yl)-2H-1,2-benzothiazin-3-carboxamid-1,1-dioxid,

22,0 g 4-Hydroxy-2-methyl-N-(4,6,7,8-tetrahydro-4-oxo-cyclopenta[g]-1-benzopyran-3-yl)-2H.1,2.benzothiazin-3-carboxamid-1,1-dioxid,

21,3 g 4-Hydroxy-2-methyl-N-(6,7-dimethyl-4-oxo-4H-1-benzopyran-3-yl)-2H-1,3-benzothiazin-3-carboxamid-1,1-dioxid,

21,4 g 4-Hydroxy-2-methyl-N-(6-methoxy-4-oxo-4H-1-benzopyran-3-yl)-2H-1,2-benzothiazin-3-carboxamid-1,-dioxid,

21,3 g 4-Hydroxy-2-methyl-N-(5,7-dimethyl-4-oxo-4H-1-benzopyran-3-yl)-2H-1,2-benzothiazin-3-carboxamid-1,1-dioxid und

20,6 g (4-Hydroxy-2-methyl-N-(8-methyl-4-oxo-4H-1-benzopyran-3-yl)-2H-1,2-benzothiazin-3-carboxamid-1,1-dioxid,

16,8 g 4-Hydroxy-2-methyl-N-(2-thiazolyl)-2H-1,2-benzothiazin-3-carboxamid-1,1-dioxid,

16,1 g 4-Hydroxy-2-methyl-N-(2-pyridyl)-2H-1,2-benzothiazin-3-carboxamid-1,1-dioxid bzw.

16,7g 4-Hydroxy-2-methyl-N-(5-methyl-3-isoxazolyl)-2H-1,2-benzothiazin-3-carboxamid-1,1-dioxid,

mit jeweils 11,9 g Diäthanolamin

das Diäthanolammoniumsalz von 4-Hydroxy-2-methyl-N-(4-oxo-4H-1-benzopyran-3-yl)-2H-1,2-benzothiazin-3-carboxamid-1,1-dioxid,

das Diäthanolammoniumsalz von 4-Hydroxy-2-methyl-N-(4,6,7,8-tetrahydro-4-oxo-cyclopenta[g]-1-benzopyran-3-yl)-2H-1,2-benzothiazin-3-carboxamid-1,1-dioxid,

das Diäthanolammoniumsalz von 4-Hydroxy-2-methyl-N-(6,7-dimethyl-4-oxo-4H-1-benzopyran-3-yl)-2H-1,2-benzothiazin-3-carboxamid-1,1-dioxid,

das Diäthanolammoniumsalz von 4-Hydroxy-2-methyl-N-(6-methoxy-4-oxo-4H-1-benzopyran-3-yl)-2H-1,2-benzothiazin-3-carboxamid-1,1-dioxid,

das Diäthanolammoniumsalz von 4-Hydroxy-2-methyl-N-(5,7-dimethyl-4-oxo-4H-1-benzopyran-3-yl)-2H-1,2-benzothiazin-3-carboxamid-1,1-dioxid,

und

das Diäthanolammoniumsalz von 4-Hydroxy-2-methyl-N-(8-methyl-4-oxo-4H-1-benzopyran-3-yl)-2H-1,2-benzothiazin-3-carboxamid-1,1-dioxid,
das Diäthanolammoniumsalz von 4-Hydroxy-2-methyl-N-(2-thiazolyl)-2H-1,2-benzothiazin-3-carboxamid-1,1-dioxid,
das Diäthanolammoniumsalz von 4-Hydroxy-2-methyl-N-(2-pyridyl)-2H-1,2-benzothiazin-3-carboxamid-1,1-dioxid,
das Diäthanolammoniumsalz von 4-Hydroxy-2-methyl-N-(5-methyl-3-isoxazolyl)-2H-1,2-benzothiazin-3-carboxamid-1,1-dioxid.

Beispiel 4: Tabletten enthaltend 25 mg Triäthanolammoniumsalz von 4-Hydroxy-2-methyl-N-(4-oxo-4H-1-benzopyran-3-yl)-2H-1,2-benzothiazin-3-carboxamid-1,1-dioxid (Wirkstoff) können z.B. wie folgt hergestellt werden:

Zusammensetzung: (für 10'000 Tabletten):

| | |
|---|---|
| Wirkstoff | 250 g |
| Lactose | 460 g |
| Maisstärke | 650 g |
| Polyvinylpyrrolidon | 20 g |
| Magnesiumstearat | 10 g |
| kolloidales Siliciumoxid | 10 g |
| Wasser | q.s. |

Der Wirkstoff, die Lactose und 450 g der Maisstärke werden gemischt und mit einer wässrigen Lösung von Polyvinylpyrrolidon befeuchtet. Das Gemisch wird granuliert und getrocknet, und mit dem Magnesiumstearat, dem kolloidalen Siliciumoxid und dem Rest der Maisstärke versetzt. Das Gemisch wird durch ein Sieb getrieben, gemischt und zu Tabletten von 140 mg Gewicht (Durchmesser: 7 mm) verpresst.

Beispiel 5: In analoger Weise wie in Beispiel 4 beschrieben, können auch Tabletten enthaltend jeweils 25 mg einer anderen Verbindung gemäss den Beispielen 1-3 hergestellt werden.

Patentansprüche (für alle benannten Länder ausser Oesterreich)

1. Ammoniumsalze substituierter 3,4-Dihydro-2H-1,2-benzothiazin-1,1-dioxide der Formel I

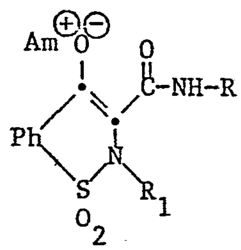

(I),

worin Ph einen gegebenenfalls substituierten 1,2-Phenylenrest bedeutet, R einen gegebenenfalls substituierten Heteroarylrest darstellt, $R_1$ Wasserstoff oder einen gegebenenfalls substituierten Kohlenwasserstoffrest aliphatischen Charakters bedeutet und $Am^{\oplus}$ das von einem pharmazeutisch verwendbaren Diniederalkyl-, Di-(hydroxyniederalkyl)- oder Tri-(hydroxyniederalkyl)-amin abgeleitete Ammonium-Kation darstellt.

2. Verbindungen gemäss Anspruch 1, mit der Massgabe, dass R von gegebenenfalls in 3- und/oder 4-Stellung durch Alkyl substituiertem 5-Isoxazolyl verschieden ist, wenn Ph unsubstituiertes 1,2-Phenylen, $R_1$ Wasserstoff oder Alkyl und $Am^{\oplus}$ Triäthanolammonium darstellt.

3. Verbindungen gemäss Anspruch 1 oder 2 der Formel I, worin Ph einen gegebenenfalls durch Niederalkyl, Niederalkoxy, Niederalkanoyl, Halogen, Nitro und/oder Trifluormethyl substituierten, 1,2-Phenylenrest bedeutet, R ein gegebenenfalls durch Niederalkyl, Niederalkoxy, Halogen, Niederalkanoyloxy, Hydroxy, Benzoyloxyreste, gegebenenfalls durch Niederalkyl oder Niederalkanoyl substituiertes Amino substituierter, als Heteroatom(e) ein Sauerstoffatom, ein Sauerstoff- oder Schwefelatom sowie ein Stickstoffatom oder ein Schwefelatom sowie zwei Stickstoffatome aufweisender 5-gliedriger oder als Heteroatom(e)

ein, zwei oder drei Stickstoffatome aufweisender 6-gliedriger monocyclischer Heteroarylrest oder ein als Heteroatom ein Sauerstoffatom aufweisender 10-gliedriger, aus zwei 6-Ringen aufgebauter, im sauerstoffhaltigen Ring Oxo aufweisender, gegebenenfalls durch Niederalkyl, Niederalkoxy, Halogen, Niederalkanoyloxy, Hydroxy, Benzoyloxyreste, gegebenenfalls durch Niederalkyl oder Niederalkanoyl substituiertes Amino und/oder an zwei benachbarten Kohlenstoffatomen im carbocyclischen Ring durch Niederalkylen oder Niederalkylendioxy substituierter bicyclischer Heteroarylrest ist und $R_1$ Wasserstoff, Niederalkyl, Niederalkenyl oder einen Phenylniederalkylrest darstellt, wobei in Benzoyl- und Phenylniederalkylgruppen das Phenyl durch Niederalkyl, Niederalkoxy, Halogen, Trifluormethyl und/oder Nitro substituiert sein kann.

4. Verbindungen gemäss Anspruch 1 der Formel I, worin Ph einen unsubstituierten oder durch Niederalkyl mit bis zu 4 Kohlenstoffatomen, Niederalkoxy mit bis zu 4 Kohlenstoffatomen, Halogen bis Atomnummer 35, Nitro oder Trifluormethyl substituierten 1,2-Phenylenrest bedeutet, R Furyl, Isoxazolyl, Thiazolyl, Isothiazolyl, Thiadiazolyl, Pyridyl, Pyrazinyl, Pyrimidinyl, Pyridazinyl oder 4-Oxo-4H- bzw. 2-Oxo-2H-1-benzopyranyl bedeutet, welches unsubstituiert oder durch Niederalkyl mit bis zu 4 Kohlenstoffatomen, Niederalkyl mit bis zu 4 Kohlenstoffatomen, Halogen bis Atomnummer 35, Niederalkanoyloxy mit bis zu 4 Kohlenstoffatomen, Hydoxy, Amino, N-Mono- oder N,N-diniederalkylamino mit bis zu 4 Kohlenstoffatomen im Alkylteil, Niederalkanoylamino mit bis zu 4 Kohlenstoffatomen und/oder, im Falle von 4-Oxo-4H- bzw- 2-Oxo-2H-1-benzopyranyl, an zwei benachbarte Kohlenstoffatome durch Niederalkylen mit bis zu 4 Kohlenstoffatomen oder Niederalkylendioxy mit bis zu 4 Kohlenstoffatomen substituiert sein kann, und $R_1$ Wasserstoff oder Niederalkyl mit bis zu 4 Kohlenstoffatomen bedeutet.

5. Verbindungen gemäss Anspruch 1 oder 2 der Formel I, worin Ph gegebenenfalls durch Niederalkyl mit bis zu 4 Kohlenstoffatomen, Niederalkoxy mit bis zu 4 Kohlenstoffatomen, Halogen bis Atomnummer 35, Trifluormethyl oder Nitro monosubstituiertes 1,2-Phenylen bedeutet, $R_1$ Wasserstoff oder Niederalkyl mit bis zu 4 Kohlenstoffatomen bedeutet, und R Furyl, gegebenenfalls in 3- und/oder 4- bzw- 4- und/ oder 5-Stellung durch Niederalkyl mit bis und mit 4 C-Atomen mono- oder disubstituiertes Isoxazolyl, durch in 4- und/oder 5-Stellung Niederalkyl mit bis und mit 4 C-Atomen substituiertes Thiazolyl, Isothiazolyl, gegebenenfalls in 5-Stellung durch Niederalkyl mit bis und mit 4 C-Atomen substituiertes Thiadiazolyl, gegebenenfalls in 3-, 4-, 5-, 6- oder 4- und 5-Stellung durch Niederalkyl mit bis und mit 4 C-Atomen, oder in 5-Stellung durch Halogen der Atomnummer bis und mit 35 oder Hydroxy substituiertes Pyridyl, Pyrazinyl, gegebenenfalls durch Niederalkyl oder Niederalkoxy mit bis und mit 4 C-Atomen, oder gegebenenfalls in 5-Stellung durch Niederalkyl oder in 6-Stellung durch Niederalkoxy mit jeweils bis und mit 4 C-Atomen substituiertes Pyridazinyl, gegebenenfalls in 4- und/oder 5-Stellung durch Niederalkyl mit bis und mit 4 C-Atomen substituiertes Pyrimdinyl, gegebenenfalls in 3- und/oder 4-Stellung durch Niederalkyl und/oder Niederalkoxy mit bis und mit 4 C-Atomen, 3- oder 4-gliedriges Niederalkylen bzw. Niederalkylendioxy substituiertes 2-Oxo-2H-1-benzopyran-7-yl oder gegebenenfalls im Pyranteil durch Niederalkyl mit bis und mit 4 C-Atomen und/oder im Benzoteil durch Halogen der Atomnummer bis und mit 35, Niederalkyl und/oder Niederalkoxy mit bis und mit 4 C-Atomen oder 3- oder 4-gliedriges Niederalkylen bzw. Niederalkylendioxy substituiertes 4-Oxo-4H-1-benzopyran-3-yl bedeutet.

6. Verbindungen gemäss Anspruch 1 der Formel I, worin $R_1$ Niederalkyl mit bis zu 4 Kohlenstoffatomen, wie Methyl, bedeutet, Ph unsubstituiertes 1,2-Phenylen bedeutet, und R im Benzoteil gegebenenfalls durch Niederalkyl mit bis zu 4 Kohlenstoffatomen, Niederalkoxy mit bis und mit 4 C-Atomen und/oder Halogen der Atomnummer bis und mit 35 mono-

oder disubstituiertes oder an zwei benachbarte C-Atome gebundenes 3- bis 4-gliedriges Niederalkylen mit bis zu 4 Kohlenstoffatomen substituiertes 4-Oxo-4H-1-benzopyran-3-yl bedeutet.

7. Verbindungen gemäss Anspruch 1 der Formel I, worin $R_1$ Niederalkyl mit bis zu 4 Kohlenstoffatomen, wie Methyl, bedeutet, $R_3$ Wasserstoff, Ph unsubstituiertes 1,2-Phenylen bedeutet und R 2-Thiazolyl, 5-Methyl-3-Isoxazolyl oder 2-Pyridyl darstellt.

8. Verbindungen gemäss einem der Ansprüche 1-7, worin $Am^{\oplus}$ Diäthylammonium, Diäthanolammonium oder Triäthanolammonium bedeutet.

9. Das Triäthanolammoniumsalz von 4-Hydroxy-2-methyl-N-(4,6,7,8-tetrahydro-cyclopenta[g]-1-benzopyran-3-yl)-2H-1,2-benzothiazin-3-carboxamid-1,1-dioxid.

10. Das Triäthanolammoniumsalz von 4-Hydroxy-2-methyl-N-(4-oxo-6,7-dimethyl-4H-1-benzopyran-3-yl)-2H-1,2-benzothiazin-3-carboxamid-1,1-dioxid.

11. Das Triäthanolammoniumsalz von 4-Hydroxy-2-methyl-N-(4-oxo-4H-1-benzopyran-3-yl)-2H-1,2-benzothiazin-3-carboxamid-1,1-dioxid.

12. Das Triäthanolammoniumsalz von 4-Hydroxy-2-methyl-N-(4-oxo-5,7-dimethyl-4H-1-benzopyran-3-yl)-2H-1,2-benzothiazin-3-carboxamid-1,1-dioxid.

13. Das Triäthanolammoniumsalz von 4-Hydroxy-2-methyl-N-(2-pyridyl)-2H-1,2-benzothiazin-3-carboxamid-1,1-dioxid.

14. Das Triäthanolammoniumsalz von 4-Hydroxy-2-methyl-N-(5-methyl-3-isoxazol-yl)-2H-1,2-benzothiazin-3-carboxamid-1,1-dioxid.

15. Das Diäthylammoniumsalz von 4-Hydroxy-2-methyl-N-(4-oxo-4H-1-benzopyran-3-yl)-2H-1,2-benzothiazin-3-carboxamid-1,1-dioxid.

16. Das Diäthylammoniumsalz von 4-Hydroxy-2-methyl-N-(2-pyridyl)-2H-1,2-benzothiazin-3-carboxamid-1,1-dioxid.

17. Das Diäthylammoniumsalz von 4-Hydroxy-2-methyl-N-(5-methyl-3-isoxazolyl)-2H-1,2-benzothiazin-3-carboxamid-1,1-dioxid.

18. Das Diäthanolammoniumsalz von 4-Hydroxy-2-methyl-N-(4-oxo-4H-1-benzopyran-3-yl)-2H-1,2-benzothiazin-3-carboxamid-1,1-dioxid.

19. Das Diäthanolammoniumsalz von 4-Hydroxy-2-methyl-N-(2-pyridyl)-2H-1,2-benzothiazin-3-carboxamid-1,1-dioxid.

20. Das Diäthanolammoniumsalz von 4-Hydroxy-2-methyl-N-(5-methyl-3-isoxazolyl)-2H-1,2-benzothiazin-3-carboxamid-1,1-dioxid.

21. Eine Verbindung gemäss einem der Ansprüche 1-45 zur Anwendung in einem Verfahren zur therapeutischen Behandlung des menschlichen oder tierischen Körpers.

22. Eine Verbindung gemäss einem der Ansprüche 1-45 als antiinflammatorisches Mittel.

23. Pharmazeutisches Präparat enthaltend mindestens eine Verbindung gemäss einem der Ansprüche 1-21 neben üblichen pharmazeutischen Hilfs- und Trägerstoffen.

24. Verfahren zur Herstellung von Ammoniumsalzen substituierter 3,4-Dihydro-2H-1,2-benzothiazin-1,1-dioxiden der Formel

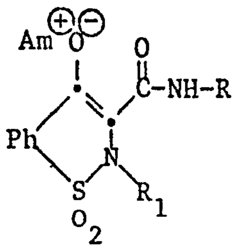

(I),

worin Ph einen gegebenenfalls substituierten 1,2-Phenylenrest bedeutet, R einen gegebenenfalls substituierten Heteroarylrest darstellt, $R_1$ Wasserstoff oder einen gegebenenfalls substituierten Kohlenwasserstoffrest aliphatischen Charakters bedeutet und $Am^{\oplus}$ das von einem pharmazeutisch verwendbaren Diniederalkyl-, Di-(hydroxy)-niederalkyl- oder Tri-(hydroxyniederalkyl)-amin Am-H abgeleitetes Ammonium-Kation darstellt, dadurch gekennzeichnet, dass man eine Verbindung der Formel II

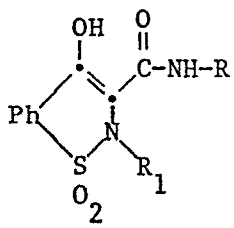

(II)

oder ein Salz davon mit einem Diniederalkyl-, Di-(hydroxyniederalkyl)- oder Tri-(hydroxyniederalkyl)-amin der Formel Am-H (III) oder einem Salz davon umsetzt.

25. Die nach dem Verfahren des Anspruch 24 erhältlichen Verbindungen.

26. Verwendung von Verbindungen gemäss einem der Ansprüche 1-25 als Arzneimittelwirkstoff oder zur Herstellung eines Arzneimittels auf nicht-chemischem Wege.

Patentansprüche (für Oesterreich)

1. Verfahren zur Herstellung von Ammoniumsalzen substituierter 3,4-Dihydro-2H-1,2-benzothiazin-1,1-dioxide der Formel

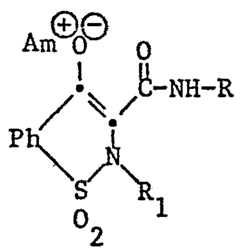

(I),

worin Ph einen gegebenenfalls substituierten 1,2-Phenylenrest bedeutet, R einen gegebenenfalls substituierten Heteroarylrest darstellt, $R_1$ Wasserstoff oder einen gegebenenfalls substituierten Kohlenwasserstoffrest aliphatischen Charakters bedeutet und $Am^{\oplus}$ das von einem pharmazeutisch verwendbaren Diniederalkyl-, Di-(hydroxy)-niederalkyl- oder Tri-(hydroxyniederalkyl)-amin Am-H abgeleitete Ammonium-Kation darstellt, dadurch gekennzeichnet, dass man eine Verbindung der Formel II

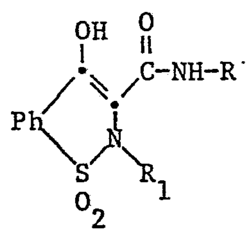

(II)

oder ein Salz davon mit einem Diniederalkyl-, Di-(hydroxyniederalkyl)- oder Tri-(hydroxyniederalkyl)-amin der Formel Am-H (III) oder einem Salz davon umsetzt.

2. Verfahren gemäss Anspruch 1 zur Herstellung von Verbindungen der Formel I, worin R einen gegebenenfalls substituierten, als Heteroatom(e) ein Sauerstoffatom, ein Sauerstoff- oder Schwefelatom sowie ein Stickstoffatom oder ein Schwefelatom sowie zwei Stickstoffatome aufweisender

5-gliedriger oder als Heteroatom(e) ein, zwei oder drei Stickstoffatome aufweisender 6-gliedriger monocyclischer Heteroarylrest oder einen im das Sauerstoffatom aufweisenden Ring durch Oxo und gegebenenfalls zusätzlich substituierten, als Heteroatom ein Sauerstoffatom aufweisender 10-gliedriger, aus 6 Ringen aufgebauter bicyclischer Heteroarylrest bedeutet, mit der Massgabe, dass R von gegebenenfalls in 3- und/oder 4-Stellung durch Alkyl substituiertem 5-Isoxazolyl verschieden ist, wenn Ph unsubstituiertes 1,2-Phenylen, $R_1$ Wasserstoff oder Alkyl und $Am^{\oplus}$ Triäthanolammonium darstellt.

3. Verfahren gemäss Anspruch 1 oder 2 zur Herstellung von Verbindungen der Formel I, worin Ph einen gegebenenfalls durch Niederalkyl, Niederalkoxy, Niederalkanoyl, Halogen, Nitro und/oder Trifluormethyl substituierten, 1,2-Phenylenrest bedeutet, R ein gegebenenfalls durch Niederalkyl, Niederalkoxy, Halogen, Niederalkanoyloxy, Hydroxy, Benzoyloxyreste, gegebenenfalls durch Niederalkyl oder Niederalkanoyl substituiertes Amino substituierter, als Heteroatom(e) ein Sauerstoffatom, ein Sauerstoff- oder Schwefelatom sowie ein Stickstoffatom oder ein Schwefelatom sowie zwei Stickstoffatome aufweisender 5-gliedriger oder als Heteroatom(e) ein, zwei oder drei Stickstoffatome aufweisender 6-gliedriger monocyclischer Heteroarylrest oder ein als Heteroatom ein Sauerstoffatom aufweisender 10-gliedriger, aus zwei 6-Ringen aufgebauter, im sauerstoffhaltigen Ring Oxo aufweisender, gegebenenfalls durch Niederalkyl, Niederalkoxy, Halogen, Niederalkanoyloxy, Hydroxy, Benzoyloxyreste, gegebenenfalls durch Niederalkyl oder Niederalkanoyl substituiertes Amino und/oder an zwei benachbarten Kohlenstoffatomen im carbocyclischen Ring durch Niederalkylen oder Niederalkylendioxy substituierter bicyclischer Heteroarylrest ist und $R_1$ Wasserstoff, Niederalkyl, Niederalkenyl oder einen Phenylniederalkylrest darstellt, wobei in Benzoyl- und Phenylniederalkylgruppen das Phenol durch Niederalkyl, Niederalkoxy, Halogen, Trifluormethyl und/oder Nitro substituiert sein kann.

4. Verfahren gemäss Anspruch 1 zur Herstellung von Verbindungen der Formel I, worin Ph einen unsubstituierten oder durch Niederalkyl mit bis zu 4 Kohlenstoffatomen, Niederalkoxy mit bis zu 4 Kohlenstoffatomen, Halogen bis Atomnummer 35, Nitro oder Trifluormethyl substituierten 1,2-Phenylenrest bedeutet, R Furyl, Isoxazolyl, Thiazolyl, Isothiazolyl, Thiadiazolyl, Pyridyl, Pyrazinyl, Pyrimidinyl, Pyridazinyl oder 4-Oxo-4H- bzw. 2-Oxo-2H-1-benzopyranyl bedeutet, welches unsubstituiert oder durch Niederalkyl mit bis zu 4 Kohlenstoffatomen, Niederalkyl mit bis zu 4 Kohlenstoffatomen, Halogen bis Atomnummer 35, Niederalkanoyloxy mit bis zu 4 Kohlenstoffatomen, Hydroxy, Amino, N-Mono- oder N,N-diniederalkylamino mit bis zu 4 Kohlenstoffatomen im Alkylteil, Niederalkanoylamino mit bis zu 4 Kohlenstoffatomen und/oder, im Falle von 4-Oxo-4H- bzw. 2-Oxo-2H-1-benzopyranyl, an zwei benachbarte Kohlenstoffatome durch Niederalkylen mit bis zu 4 Kohlenstoffatomen oder Niederalkylendioxy mit bis zu 4 Kohlenstoffatomen substituiert sein kann, und $R_1$ Wasserstoff oder Niederalkyl mit bis zu 4 Kohlenstoffatomen bedeutet.

5. Verfahren gemäss Anspruch 1 oder 2 zur Herstellung von Verbindungen der Formel I, worin Ph gegebenenfalls durch Niederalkyl mit bis zu 4 Kohlenstoffatomen, Niederalkoxy mit bis zu 4 Kohlenstoffatomen, Halogen bis Atomnummer 35, Trifluormethyl oder Nitro monosubstituiertes 1,2-Phenylen bedeutet, $R_1$ Wasserstoff oder Niederalkyl mit bis zu 4 Kohlenstoffatomen bedeutet, und R Furyl, gegebenenfalls in 3- und/oder 4- bzw. 4- und/oder 5-Stellung durch Niederalkyl mit bis und mit 4 C-Atomen mono- oder disubstituiertes Isoxazolyl, durch in 4- und/oder 5-Stellung Niederalkyl mit bis und mit 4 C-Atomen substituiertes Thiazolyl, Isothiazolyl, gegebenenfalls in 5-Stellung durch Niederalkyl mit bis und mit 4 C-Atomen substituiertes Thiadiazolyl, gegebenenfalls in 3-, 4-, 5-, 6- oder 4- und 5-Stellung durch Niederalkyl mit bis und mit 4 C-Atomen, oder in 5-Stellung durch Halogen der Atomnummer bis und mit 35 oder Hydroxy substituiertes Pyridyl, Pyrazinyl, gegebenenfalls durch Niederalkyl oder Niederalkoxy mit bis und mit 4 C-Atomen, oder gegebenenfalls in 5-Stellung durch Niederalkyl oder in 6-Stellung durch Niederalkoxy mit jeweils bis und mit 4 C-Atomen substituiertes

Pyridazinyl, gegebenenfalls in 4- und/oder 5-Stellung durch Niederalkyl mit bis und mit 4 C-Atomen substituiertes Pyrimdinyl, gegebenenfalls in 3- und/oder 4-Stellung durch Niederalkyl und/oder Niederalkoxy mit bis und mit 4 C-Atomen, 3- oder 4-gliedriges Niederalkylen bzw. Niederalkylendioxy substituiertes 2-Oxo-2H-1-benzopyran-7-yl oder gegebenenfalls im Pyranteil durch Niederalkyl mit bis und mit 4 C-Atomen und/oder im Benzoteil durch Halogen der Atomnummer bis und mit 35, Niederalkyl und/oder Niederalkoxy mit bis und mit 4 C-Atomen oder 3- oder 4-gliedriges Niederalkylen bzw. Niederalkylendioxy substitueirtes 4-Oxo-4H-benzopyran-3-yl bedeutet.

6. Verfahren gemäss Anspruch 1 zur Herstellung von Verbindungen der Formel I, worin $R_1$ Niederalkyl mit bis zu 4 Kohlenstoffatomen, wie Methyl, bedeutet, Ph unsubstituiertes 1,2-Phenylen bedeutet, und R im Benzoteil gegebenenfalls durch Niederalkyl mit bis zu 4 Kohlenstoffatomen, Niederalkoxy mit bis und mit 4 C-Atomen und/oder Halogen der Atomnummer bis und mit 35 mono- oder disubstituiertes oder an zwei benachbarte C-Atome gebundenes 3- bis 4-gliedriges Niederalkylen mit bis zu 4 Kohlenstoffatomen substituiertes 4-Oxo-4H-1-benzopyran-3-yl bedeutet.

7. Verbindungen gemäss Anspruch 1 zur Herstellung von Verbindungen der Formel I, worin $R_1$ Niederalkyl mit bis zu 4 Kohlenstoffatomen, wie Methyl, bedeutet, $R_3$ Wasserstoff, Ph unsubstituiertes 1,2-Phenylen bedeutet und R 2-Thiazolyl, 5-Methyl-3-Isoxazolyl oder 2-Pyridyl darstellt.

8. Verfahren gemäss einem der Ansprüche 1-7 zur Herstellung von Verbindungen der Formel I, worin $Am^{\oplus}$ Diäthylammonium, Diäthanolammonium oder Triäthanolammonium bedeutet.

9. Verfahren nach einem der Ansprüche 1-8, dadurch gekennzeichnet, dass man das Triäthanolammoniumsalz von 4-Hydroxy-2-methyl-N-(4,6,7,8-tetra-

hydro-cyclopenta[g]-1-benzopyran-3-yl)-2H-1,2-benzothiazin-3-carboxamid-1,1-dioxid herstellt.

10. Verfahren nach einem der Ansprüche 1-8, dadurch gekennzeichnet, dass man das Triäthanolammoniumsalz von 4-Hydroxy-2-methyl-N-(4-oxo-6,7-dimethyl-4H-1-benzopyran-3-yl)-2H-1,2-benzothiazin-3-carboxamid-1,1-dioxid herstellt.

11. Verfahren nach einem der Ansprüche 1-8, dadurch gekennzeichnet, dass man das Triäthanolammoniumsalz von 4-Hydroxy-2-methyl-N-(4-oxo-4H-1-benzopyran-3-yl)-2H-1,2-benzothiazin-3-carboxamid-1,1-dioxid herstellt.

12. Verfahren nach einem der Ansprüche 1-8, dadurch gekennzeichnet, dass man das Triäthylammoniumsalz von 4-Hydroxy-2-methyl-N-(4-oxo-5,7-dimethyl-4H-1-benzopyran-3-yl)-2H-1,2-benzothaizin-3-carboxamid-1,1-dioxid herstellt.

13. Verfahren nach einem der Ansprüche 1-8, dadurch gekennzeichnet, dass man das Triäthanolammoniumsalz von 4-Hydroxy-2-methyl-N-(2-pyridyl)-2H-1,2-benzothiazin-3-carboxamid-1,1-dioxid herstellt.

14. Verfahren nach einem der Ansprüche 1-8, dadurch gekennzeichnet, dass man das Triäthanolammoniumsalz von 4-Hydroxy-2-methyl-N-(3-methyl-3-isoxazol-yl)-2H-1,2-benzothiazin-3-carboxamid-1,1-dioxid herstellt.

15. Verfahren nach einem der Ansprüche 1-8, dadurch gekennzeichnet, dass man das Diäthylammoniumsalz von 4-Hydroxy-2-methyl-N-(4-oxo-4H-1-benzopyran-3-yl)-2H-1,2-benzothiazin-3-carboxamid-1,1-dioxid herstellt.

16. Verfahren nach einem der Ansprüche 1-8, dadurch gekennzeichnet, dass man das Diäthylammoniumsalz von 4-Hydroxy-2-methyl-N-(2-pyridyl)-2H-1,2-benzothiazin-3-carboxamid-1,1-dioxid herstellt.

17. Verfahren nach einem der Ansprüche 1-8, dadurch gekennzeichnet, dass man das Diäthylammoniumsalz von 4-Hydroxy-2-methyl-N-(5-methyl-3-isoxazolyl)-2H-1,2-benzothiazin-3-carboxamid-1,1-dioxid herstellt.

18. Verfahren nach einem der Ansprüche 1-8, dadurch gekennzeichnet, dass man das Diäthanolammoniumsalz von 4-Hydroxy-2-methyl-N-(4-oxo-4H-1-benzopyran-3-yl)-2H-1,2-benzothiazin-3-carboxamid-1,1-dioxid herstellt.

19. Verfahren nach einem der Ansprüche 1-8, dadurch gekennzeichnet, dass man das Diäthanolammoniumsalz von 4-Hydroxy-2-methyl-N-(2-pyridyl)-2H-1,2-benzothiazin-3-carboxamid-1,1-dioxid herstellt.

20. Verfahren nach einem der Ansprüche 1-8, dadurch gekennzeichnet, dass man das Diäthanolammoniumsalz von 4-Hydroxy-2-methyl-N-(5-methyl-3-isoxazolyl)-2H-1,2-benzothiazin-3-carboxamid-1,1-dioxid herstellt.

21. Die nach dem Verfahren des Anspruchs 1 erhältlichen Verbindungen.

22. Das Verfahren zur Herstellung von pharmazeutischen Präparaten, dadurch gekennzeichnet, dass man eine Verbindung gemäss einem der Ansprüche 1-20 mit üblichen pharmazeutischen Hilfs- und/oder Trägerstoffen vermischt.

Europäisches Patentamt

# EUROPÄISCHER RECHERCHENBERICHT

0082217

Nummer der Anmeldung

EP 81 11 0735.8

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.3) |
|---|---|---|---|
| A | US - A - 3 992 535 (BOEHRINGER INGELHEM)<br>* Ansprüche 1, 5, 8; Spalte 33, Beispiel 61 *<br>-- | 1,23 | C 07 D 417/12<br>A 61 K 31/54 |
| A | EP - A1 - 0 003 360 (CIBA-GEIGY)<br>* Ansprüche 1, 8; Seite 4, Zeilen 22 bis 33 * | 1,23 | |
| D | & US - A - 4 213 980<br>-- | | |
| D,A | US - A - 3 822 258 (WARNER-LAMBERT CO.)<br>* Anspruch 1; Spalte 1, Zeilen 49 bis 54 *<br>---- | 1,23 | RECHERCHIERTE SACHGEBIETE (Int. Cl.3)<br>A 61 K 31/54<br>C 07 D 279/02<br>C 07 D 417/12 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 30-07-1982 | PHILLIPS |